# FASCICULE DE BREVET EUROPEEN

(11) **EP 1 977 780 B1**
(45) Date de publication et mention de la délivrance du brevet: **04.10.2017**
(21) Numéro de dépôt: 08290230.5
(22) Date de dépôt: 11.03.2008
(51) Int. Cl.: A61M 16/04, A61M 16/08, A61M 16/20

(54) **Ensemble respiratoire à respirateur artificiel et sonde**
Beatmungseinheit mit künstlichem Beatmungsgerät und Sonde
Breathing apparatus with artificial respirator and probe

(30) Priorité: 02.04.2007 FR 0702384
(43) Date de publication de la demande: 08.10.2008
(73) Titulaire: Boussignac, Georges, 92160 Antony (FR)
(72) Inventeur: Boussignac, Georges, 92160 Antony (FR)
(74) Mandataire: Jacobacci Coralis Harle

(56) Documents cités:
- EP-A- 0 747 077
- WO-A-01/24861
- WO-A-02/094360
- WO-A-2006/090043
- US-A- 3 815 606
- US-A- 4 265 235
- US-A- 6 155 252

## Description

La présente invention concerne un ensemble respiratoire comportant un respirateur artificiel et une sonde respiratoire, sonde- dont l'extrémité distale est destinée à être introduite dans l'appareil respiratoire d'un patient et dont l'extrémité proximale est reliée à la sortie dudit respirateur artificiel engendrant des impulsions de gaz respiratoire, correspondant à des inspirations pour ledit patient, en réponse aux impulsions de gaz respiratoire pollué expirées par le patient.

Dans les ensembles respiratoires de ce type, le gaz respiratoire frais et le gaz respiratoire pollué (chargé de gaz carbonique) circulent en alternance dans la sonde, mais en sens opposés. Par ailleurs, il est courant que, lors des expirations, la totalité du gaz respiratoire pollué ne soit pas évacué. Il en résulte donc que ce gaz respiratoire pollué non évacué s'oppose à l'introduction consécutive du gaz respiratoire frais, ce qui a pour conséquence une mauvaise oxygénation du patient. Pour tenter d'éviter cet inconvénient, on augmente alors la pression sous laquelle ledit respirateur artificiel délivre le gaz respiratoire frais, pour aider à chasser le gaz respiratoire pollué. Mais alors, on risque de blesser le patient, surtout si celui-ci est un enfant. Les documents US 6 155 252 A, US 3 815 606 A, WO 02/094360 A, WO 2006/090043 A et WO 01/24861 A décrivent des ensembles respiratoires de l'art antérieur.

La présente invention a pour objet de remédier aux inconvénients mentionnés ci-dessus. A cette fin, selon l'invention, l'ensemble respiratoire comportant :
- un respirateur artificiel pourvu d'une sortie par laquelle ledit respirateur artificiel :
   - reçoit des impulsions de gaz respiratoire pollué correspondant à des expirations d'un patient, et
   - en réponse auxdites impulsions de gaz respiratoire pollué, émet des impulsions de gaz respiratoire frais correspondant à des inspirations dudit patinent ; et
- une sonde respiratoire dont l'extrémité distale est destinée à être introduite dans l'appareil respiratoire dudit patient et dont l'extrémité proximale est reliée à ladite sortie dudit respirateur artificiel,
est remarquable en ce que :
- ladite sonde comporte deux voies indépendantes dont :
   - les extrémités distales sont destinées à être introduites en commun dans ledit appareil respiratoire du patient, et
   - les extrémités proximales sont reliées en commun à ladite sortie dudit respirateur artificiel par l'intermédiaire de valves unidirectionnelles respectives ; et
- l'une desdites valves unidirectionnelles est passante de l'extrémité proximale vers l'extrémité distale de la voie à laquelle elle est reliée, tandis que l'autre desdites valves unidirectionnelles est passante de l'extrémité distale vers l'extrémité proximale de l'autre desdites voies à laquelle elle est reliée.

Ainsi, grâce à la présente invention, le gaz respiratoire pollué résiduel ne peut pas s'opposer à l'introduction du gaz respiratoire frais à travers la voie respiratoire pourvue de la valve unidirectionnelle passante de l'extrémité proximale vers l'extrémité distale. De plus, rien ne s'oppose à l'évacuation, à travers l'autre voie respiratoire, de l'éventuel gaz résiduel pollué sous l'action du gaz respiratoire frais introduit. Il n'y a donc aucune nécessité à injecter le gaz respiratoire sous une pression excessive.

Lesdites voies de la sonde peuvent être disposées en parallèle ou bien coaxialement.

Dans ce dernier cas, la sonde peut comporter une voie centrale constituée par un tube souple et une voie périphérique constituée par une gaine souple entourant ledit tube souple. Dans un mode de réalisation avantageux, l'extrémité distale de la gaine souple peut être solidarisée de l'extrémité distale dudit tube souple et ce dernier peut-comporter, au voisinage de son extrémité distale, au moins un passage traversant disposé à l'intérieur de ladite gaine souple.

La sonde peut être du type buccal ou nasal.

Les figures du dessin annexé feront bien comprendre comment l'invention peut être réalisée. Sur ces figures, des références identiques désignent des éléments semblables.
La figure 1 illustre, en coupe longitudinale schématique, un premier mode de réalisation conforme à la présente invention.
La figure 2 illustre l'implantation, dans un patient, de la sonde de la figure 1, lorsque ladite sonde est du type nasal.
Les figures 3 et 5 illustrent respectivement, en coupe longitudinale schématique, deux autres modes de réalisation conformes à la présente invention.
La figure 4 illustre l'implantation, dans un patient, de la sonde de la figure 3 lorsque ladite sonde est du type buccal.

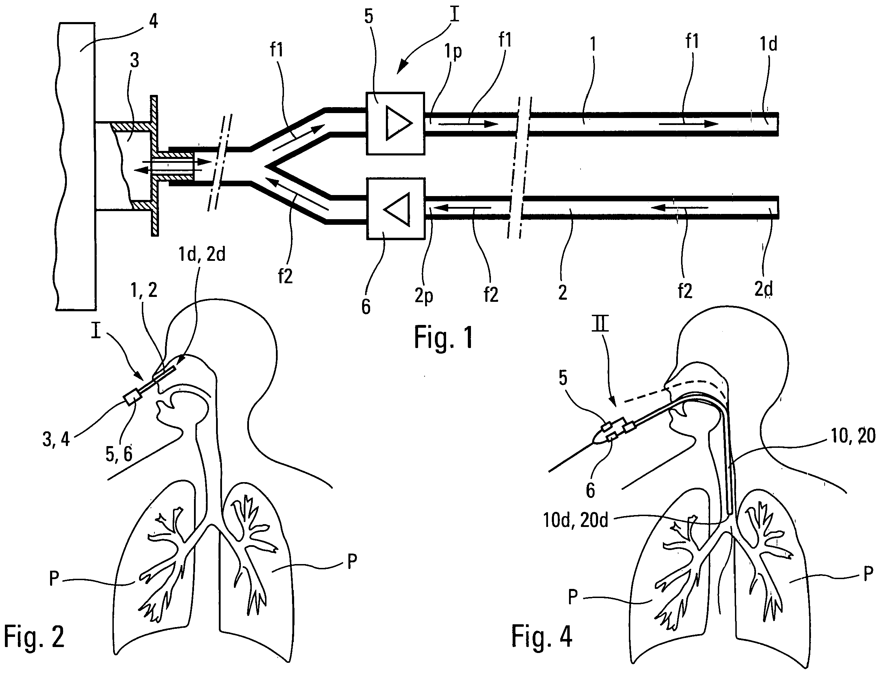

La sonde respiratoire I, représentée sur la figure 1, comporte deux voies respiratoires indépendantes, en parallèle, respectivement constituées par des tubes souples 1 et 2.

Du côté proximal, lesdits tubes souples 1 et 2 sont reliés en commun à la sortie 3 d'un respirateur artificiel 4 (très partiellement représenté) par l'intermédiaire de valves unidirectionnelles respectives 5 et 6. La valve 5, montée sur le tube souple 1, est passante de l'extrémité proximale 1p vers l'extrémité distale 1d dudit tube souple 1 et bloquante dans le sens inverse. Au contraire, la valve 6, montée sur le tube souple 2, est passouple 2 et bloquante dans le sens inverse.

Bien que la sonde I puisse être du type buccal, elle est particulièrement appropriée à être utilisée comme sonde nasale, comme cela est illustré schématiquement sur la figure 2. Sur cette figure, on a représenté schématiquement les poumons P d'un patient, les extrémités distales 1d et 2d desdits tubes 1 et 2 étant respectivement introduites dans les narines dudit patient.

Ainsi, quand le respirateur artificiel 4 adresse à la sonde I une impulsion de gaz respiratoire correspondant à une inspiration pour ledit patient, cette impulsion est transmise auxdits poumons P, à travers la valve 5 et le tube 1, comme cela est illustré par les flèches f1 sur la figure 1.

En revanche, le gaz respiratoire pollué, correspondant à une précédente impulsion de gaz et se trouvant dans les poumons P, est chassé hors de ceux-ci et est adressé au respirateur artificiel 4 à travers le tube 2 et la valve 6, comme cela est illustré par les flèches f2 sur la figure 1. Le respirateur artificiel 4 détecte l'arrivée de ce gaz respiratoire pollué correspondant à une expiration du patient et peut adresser une nouvelle impulsion de gaz respiratoire, correspondant à une inspiration, auxdits poumons P.

On conçoit aisément que, grâce à ladite sonde I, l'introduction de gaz respiratoire dans les poumons P d'un patient ne peut être gênée par le gaz respiratoire pollué se trouvant dans lesdits poumons et que, au contraire, le gaz respiratoire pollué est éliminé hors de ceux-ci sans aucune difficulté.

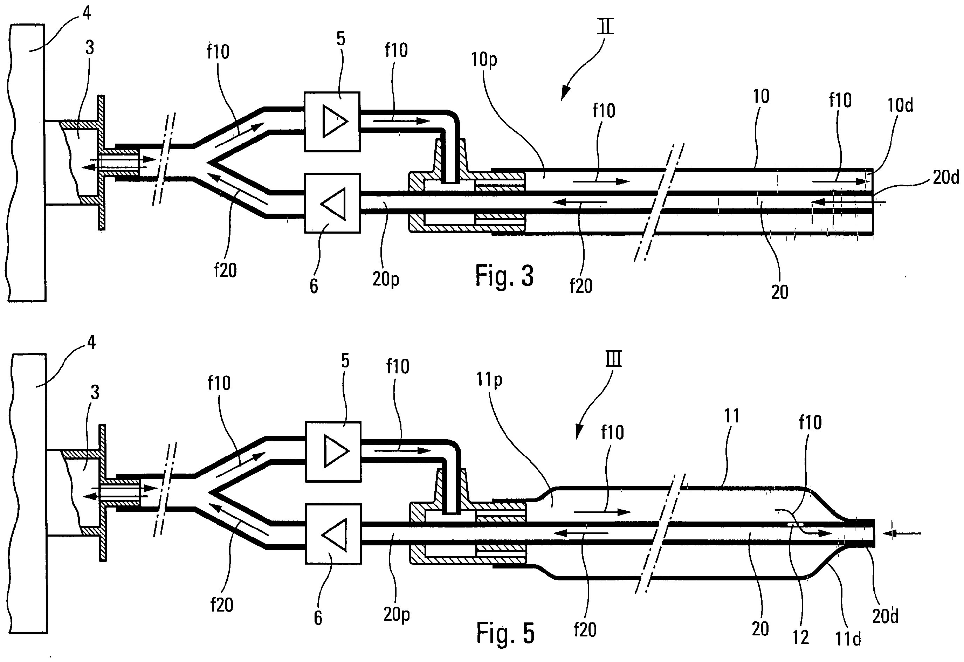

Sur la figure 3, on a représenté une sonde II, dans laquelle les deux voies respiratoires indépendantes, respectivement formées par des tubes souples 10 et 20, sont coaxiales, au lieu d'être parallèles comme les voies 1 et 2 de la sonde I. Sur cette la figure 1 et auquel sont reliées les extrémités proximales 10p et 20p desdits tubes souples 10 et 20.

La sonde II pourrait être de type nasal ; toutefois elle est avantageusement de type buccal, comme cela est illustré sur la figure 4. En effet, dans ce cas, elle peut être introduite dans l'appareil respiratoire du patient, jusqu'à ce que les extrémités distales 10d et 20d des tubes souples coaxiaux 10 et 20 se trouvent au voisinage de la carène C. Ainsi, l'espace mort entre l'extrémité distale de la sonde II et les poumons P est réduit à un minimum.

Ainsi, quand le respirateur artificiel 4 adresse à la sonde II une impulsion de gaz respiratoire correspondant à une inspiration pour le patient, cette impulsion est transmise à la carène C, à travers la valve 5 et le tube périphérique 10, comme cela est illustré par les flèches f10 sur la figure 3.

En revanche, le gaz respiratoire pollué se trouvant dans les poumons P est chassé hors de ceux-ci, à partir de la carène C et à travers le tube central 20, comme cela est illustré par les flèches f20 sur la figure 3. Une nouvelle impulsion de gaz respiratoire peut alors être adressée par le respirateur artificiel 4 (flèches f10) aux poumons P.

Sur la figure 5, on a représenté une variante de réalisation III de la sonde II de la figure 3. Dans la sonde III, on retrouve l'agencement 3, 4, 5, 6 et 20 décrit ci-dessus. En revanche, le tube périphérique 10 est remplacé par une gaine souple 11 qui entoure le tube central 20 et dont l'extrémité proximale 11p est reliée à la valve 5. L'extrémité distale 11d de la gaine souple 11 est solidarisée de façon étanche de l'extrémité distale 20d et ledit tube 20 comporte, au voisinage de son extrémité distale 20d, un passage traversant 12, se trouvant à l'intérieur de la gaine souple 11. Dans ce cas, les impulsions de gaz respiratoire (flèches f10) engendrées par le respirateur artificiel 4, traversent le passage 12 et sont adressées aux poumons à travers l'extrémité distale 20d du tube central 20. On comprendra aisément que la sonde III peut être utilisée de façon analogue à la sonde II, comme cela est illustré par la figure 4.

## Revendications

1. Ensemble respiratoire comportant :
- un respirateur artificiel (4) pourvu d'une sortie (3) par laquelle ledit respirateur artificiel (4) :
. reçoit des impulsions de gaz respiratoire pollué correspondant à des expirations d'un patient, et
. en réponse auxdites impulsions de gaz respiratoire pollué, émet des impulsions de gaz respiratoire frais correspondant à des inspirations dudit patient ; et
- une sonde respiratoire dont l'extrémité distale est destinée à être introduite dans l'appareil respiratoire dudit patient et dont l'extrémité proximale est reliée à ladite sortie (3) dudit respirateur artificiel (4), **caractérisé en ce que** :
- ladite sonde (I, II,III) comporte deux voies indépendantes (1, 2 - 10, 20 - 11, 20) dont :
. les extrémités distales sont destinées à être introduites en commun dans ledit appareil respiratoire du patient, et
. les extrémités proximales sont reliées en commun à ladite sortie (3) dudit respirateur artificiel (4) par l'intermédiaire de valves unidirectionnelles respectives (5,6); et
- l'une (5) desdites valves unidirectionnelles est passante de l'extrémité proximale vers l'extrémité distale de la voie à laquelle elle est reliée, tandis que l'autre (6) desdites valves unidirectionnelles est passante de l'extrémité distale vers l'extrémité proximale de l'autre desdites voies à laquelle elle est reliée.

2. Ensemble selon la revendication 1,
**caractérisé en ce que** lesdites deux voies (1, 2) de la sonde sont disposées en parallèle.

3. Ensemble selon la revendication 1,
**caractérisé en ce que** lesdites deux voies (10, 20 - 11, 20) de la sonde sont coaxiales.

4. Ensemble selon la revendication 3,
**caractérisé en ce que** ladite sonde comporte :
- une voie centrale (20) constituée par un tube souple ; et
- une voie périphérique constituée par une gaine souple (11) entourant ledit tube souple axial (20).

5. Ensemble selon la revendication 4,
**caractérisé en ce que** l'extrémité distale (11d) de ladite gaine souple (11) est solidarisée de l'extrémité distale (20d) dudit tube souple central (20) et **en ce que** ledit tube souple (20) comporte au voisinage de son extrémité distale au moins un passage traversant (12) disposé à l'intérieur de ladite gaine souple (11).

6. Ensemble selon l'une des revendications 1 à 5,
**caractérisé en ce que** ladite sonde est de type buccal.

7. Ensemble selon l'une des revendications 1 à 5,
**caractérisé en ce que** ladite sonde est de type nasal.

## Patentansprüche

1. Beatmungseinheit mit
- einem künstlichen Beatmungsgerät (4), das mit einem Ausgang (3) versehen ist, durch den das künstliche Beatmungsgerät (4)
= Impulse von verunreinigtem Atemgas empfängt, das den Ausatmungen eines Patienten entspricht, und
= als Reaktion auf die Impulse von verunreinigtem Atemgas Impulse von frischem Atemgasausgibt, die den Einatmungen des Patienten entsprechen, und
- einer Atemsonde, deren distales Ende dazu bestimmt ist, in die Atemwege des Patienten eingeführt zu werden, und deren proximales Ende mit dem Ausgang (3) des künstlichen Beatmungsgeräts (4) verbunden ist,
**dadurch gekennzeichnet, daß**
- die Sonde (I, II, III) zwei unabhängige Wege (1, 2 ; 10, 20 ; 11, 20) aufweist, deren
= distale Enden dazu bestimmt sind, gemeinsam in die Atemwege des Patienten eingeführt zu werden, und
= proximale Enden gemeinsam über jeweilige Rückschlagventile (5, 6) mit dem Ausgang (3) des künstlichen Beatmungsgeräts (4) verbunden sind, und
- eins (5) der Rückschlagventile vom proximalen Ende zum distalen Ende des Wegs durchlässig ist, mit dem es verbunden ist, während das andere (6) der Rückschlagventile vom distalen Ende zum proximalen Ende des anderen der Wege durchlässig ist, mit dem es verbunden ist.

2. Einheit gemäß Anspruch 1, **dadurch gekennzeichnet, daß** die beiden Wege (1, 2) der Sonde parallel angeordnet sind.

3. Einheit gemäß Anspruch 1, **dadurch gekennzeichnet, daß** die beiden Wege (10, 20 ; 11, 20) der Sonde koaxial sind.

4. Einheit gemäß Anspruch 3, **dadurch gekennzeichnet, daß** die Sonde
- einen durch einen flexiblen Schlauch gebildeten zentralen Weg (20) und
- einen durch einen flexiblen Umhüllungsschlauch (11) gebildeten peripheren Weg, der den axialen flexiblen Schlauch (20) umgibt,
aufweist.

5. Einheit gemäß Anspruch 4, **dadurch gekennzeichnet, daß** das distale Ende (11d) des flexiblen Umhüllungsschlauchs (11) mit dem distalen Ende (20d) des zentralen flexiblen Schlauchs (20) verbunden ist und daß der flexible Schlauch (20) im Bereich seines distalen Endes wenigstens einen Durchlaß (12) aufweist, der im Inneren des flexiblen Umhüllungsschlauchs (11) angeordnet ist.

6. Einheit gemäß einem der Ansprüche 1 bis 5, **dadurch gekennzeichnet, daß** die Sonde vom Typ einer Mundsonde ist.

7. Einheit gemäß einem der Ansprüche 1 bis 5, **dadurch gekennzeichnet, daß** die Sonde vom Typ einer Nasensonde ist.

## Claims

1. A respiratory assembly comprising:
- an artificial respirator (4) provided with an output (3) through which said artificial respirator (4):
receives pulses of polluted respiratory gas corresponding to exhalations for a patient, and
. in response to said pulses of polluted respiratory gas, emits pulses of fresh respiratory gas corresponding to inhalations for said patient; and
- a respiratory probe, whose distal end is intended to be inserted in the respiratory system of said patient and whose proximal end is connected to said output (3) of said artificial respirator (4),
**characterized in that**:
- said probe (I, II, III) comprises two independent channels (1, 2-10, 20-11, 20) of which:
. the distal ends are intended to be inserted in common in said respiratory system of said patient, and
. the proximal ends are connected in common to said output (3) of said artificial respirator (4) through respective unidirectional valves (5, 6) ; and
- one (5) of said unidirectional valves allows flow in the direction from the proximal end towards the distal end of the channel to which it is connected, whilst the other (6) of said unidirectional vales allows flow in the direction from the distal end towards the proximal end of the other one of said channels to which it is connected.

2. The respiratory assembly as claimed in claim 1, **characterized in that** said two channels (1, 2) of the probe are disposed in parallel.

3. The respiratory assembly as claimed in claim 1, **characterized in that** said two channels (10, 20-11, 20) of the probe are coaxial.

4. The respiratory assembly as claimed in claim 3, **characterized in that** said probe comprises:
- a central channel (20) constituted by a flexible tube; and
- a peripheral channel constituted by a flexible sleeve (11) surrounding said axial flexible tube (20).

5. The respiratory assembly as claimed in claim 4, **characterized in that** the distal end (11d) of said flexible sleeve (11) is firmly joined to the distal end (20d) of said central flexible tube (20) and wherein said flexible tube (20) comprises in the vicinity of its distal end at least one traversing passage (12) disposed inside said flexible sleeve (11).

6. The respiratory assembly as claimed in one of claims 1 to 5, **characterized in that** said probe is of the buccal type.

7. The respiratory assembly as claimed in one of claims 1 to 5, **characterized in that** said probe is of the nasal type.
